# EUROPEAN PATENT APPLICATION

(11) **EP 1 895 295 A2**
(43) Date of publication of application: **05.03.2008**
(21) Application number: 07250753.6
(22) Date of filing: 22.02.2007
(51) Int. Cl.: G01N 27/407, G01N 33/00

(54) **Gas sensor and method of manufacturing the same**

(30) Priority: 01.09.2006 JP 2006238220
(71) Applicant: Sumitomo Electric Industries, Ltd., Osaka-shi, Osaka 541-0041 (JP)
(72) Inventor: Ota, Nobuhiro, c/o Itami Works of, Itami-shi Hyogo 664-0016 (JP)
(74) Representative: Cross, Rupert Edward Blount

(57) **Abstract**

With a gas sensor comprising a structure including a reference pole having a reference material and an electronic conductive material and a sensing pole having a sensing material and an electronic conductive material provided through a solid electrolyte in which the said solid electrolyte, the said reference material and the said sensing material are constituted of lithium ionic conductors and a method of manufacturing the same, an equilibrium potential gas sensor employing a solid electrolyte operable at a low temperature not more than 200°C, particularly at the room temperature, not influenced by moisture or the like and excellent in age-based stability with high measurement sensitivity as well as high responsibility and a method of manufacturing the same can be provided.

## Description

### BACKGROUND OF THE INVENTION

### Field of the Invention

The present invention relates to a gas sensor and a method of manufacturing the same. More specifically, the present invention relates to an equilibrium potential gas sensor employing a solid electrolyte capable of measuring the concentration of carbon dioxide (CO₂) or the like under an atmosphere at a low temperature with high performance and with no aged deterioration and a method of manufacturing the same.

### Description of the Background Art

A gas sensor having a sensing pole (working electrode) and a reference pole (reference electrode) provided through a solid electrolyte is proposed as that for measuring the concentration of carbon dioxide (CO₂) or the like under an atmosphere. This is an equilibrium potential gas sensor measuring the concentration of the gas through electromotive force, generated between the sensing pole and the reference pole, whose quantity varies with the quantity of the gas dissolving into the sensing pole. In relation to this gas sensor, various types of preferable materials and structures have been proposed for the sensing pole, the reference pole and the solid electrolyte respectively.

For example, known is a solid electrolyte constituted of an ionic conductor such as NASICON (Na₃Zr₂Si₂PO₁₂) or Na-β-Al₂O₃ which is a sodium ionic conductor, YSZ (Y₂O₃-added ZrO₂) which is an oxygen ionic conductor or PbSnO₄ or LaF₃ which is a fluorine ionic conductor.

An attempt to form a thin film of such a solid electrolyte is also made. For example, Japanese Patent Publication No. 7-85071 (1995, Patent Document 1) discloses a thin-film carbon dioxide sensor formed by stacking a reference pole, a filmy solid electrolyte (solid electrolytic film) and a sensing pole in this order on a dense impermeable ceramic substrate. An oxygen ionic conductor is employed for the solid electrolytic film.

A lithium ionic conductor is also proposed as the material for the oxygen ionic conductor, and solid electrolytes employing dense sintered bodies of Li_{4-0.5X}Ti_{5-X}Al_{1.5X}O₁₂ and Li_{4-2X}Ti_{5-X}Mg_{3X}O₁₂ (Japanese Patent Laying-Open No. 2001-83116) (Patent Document 2) and Japanese Patent Laying-Open No. 8-189915 (1996) (Patent Document 3)), LiTi₂O₄ (Japanese Patent Laying-Open No. 2002-357586 (Patent Document 4)), LiLa₉(SiO₄)₆O₂ (Japanese Patent Laying-Open No. 2000-235017 (Patent Document 5)), Li_{1+X+Y}M_{X}Ti_{2-X}Si_{Y}P_{3-Y}O₁₂ (M = Al or Ga) (Japanese Patent No. 3012211 (Patent Document 6)) and Li₂ZrSi₆O₁₅ (Japanese Patent Laying-Open No. 2001-221771 (Patent Document 7)) are disclosed, for example.

A reference pole employing Na_{X}CoO₂, for example, is disclosed, and a carbon dioxide sensor employing this reference pole along with a solid electrolyte of NASICON and a sensing pole of a composite prepared by adding a mixture of Li₂CO₃ and BaCO₃ to ITO (indium tin oxide) is also disclosed (Electrochemistry, Vol. 71, No. 6, pp. 496 to 502 (2003) (Non-Patent Document 1)).

Also as to the sensing pole (working electrode), various techniques are disclosed such as a technique of providing two layers having different mixing ratios of electronic conductive materials, defining a mixing ratio between metal salt and an electronic conductive material having a solid electrolyte dissociation equilibrium and setting the thickness of the sensing pole to not more than 20 µm (Japanese Patent No. 3256618 (Patent Document 8)).

However, most of the generally known gas sensors must be heated to about 400 to 500°C, in order to operate. Therefore, development of a gas sensor excellently operating at a low temperature of not more than 200°C, particularly at the room temperature (25°C), has been desired.

For example, a carbon dioxide sensor employing a sensing pole of tin oxide and a reference pole of Na_{X}CoO₂ is reported as that operating at the room temperature. However, such a gas sensor generally requires water in order to sense carbon dioxide, and is disadvantageously influenced by moisture.

In relation to this, a gas sensor employing a mixture of a metal oxide such as tin oxide and a metal carbooxide or a metal carbooxyhydride for a sensing pole is developed as a moisture-resistant carbon dioxide sensor operating at the room temperature (Japanese Patent No. 3533334 (Patent Document 9) or Japanese Patent Laying-Open No. 2004-239831 (Patent Document 10)). However, such a gas sensor employing a mixture of a metal oxide such as tin oxide and a metal carbooxide or a metal carbooxyhydride for the sensing pole is still insufficient in age-based stability as well as in responsibility.

### SUMMARY OF THE INVENTION

An object of the present invention is to provide an equilibrium potential gas sensor employing a solid electrolyte operative at a low temperature of not more than 200°C, particularly at the room temperature, not influenced by moisture or the like and excellent in age-based stability with high measurement sensitivity and responsibility and a method of manufacturing the same.

In order to solve the aforementioned problems, the inventors have conducted deep studies, to lead to the following recognitions:
(1) If components such as a sensing pole and a solid electrolyte of a gas sensor are constituted of different types of ionic conductors, an ion-exchange layer is formed on the interface between the components to serve as a new source generating electromotive force, leading to noise in measurement at a low temperature of not more than 200°C.
(2) While an equilibrium potential gas sensor measures a voltage under high-input impedance of about 1 GΩ (gigaohm), direct current of about several 10 pA (picoamperes) is still inevitably generated. If ion exchange continues on the said ion-exchange layer, therefore, the exchange capacity or the thickness of the ion-change layer is aged with time to exert bad influence on age-based stability of an output in measurement of the gas concentration.

The inventors have conducted further studies on the basis of the aforementioned recognitions, to find out that a gas sensor capable of preventing formation of an ion-exchange layer, excellent in age-based stability and noiseless with high measurement sensitivity can be obtained by preparing all of a reference material contained in a reference pole, a solid electrolyte and a sensing material contained in a sensing pole from lithium ionic conductors and that this gas sensor is not influenced by moisture or the like and can obtain high responsibility and a stable output at a low temperature, and completed the present invention as follows:

The gas sensor according to the present invention comprises a structure including a reference pole having a reference material and an electronic conductive material and a sensing pole having a sensing material and an electronic conductive material provided through a solid electrolyte, while the said solid electrolyte, the said reference material and the said sensing material are constituted of lithium ionic conductors.

The inventive gas sensor having the aforementioned structure can stably sense gas such as carbon dioxide at a low temperature of not more than 200°C such as the room temperature, for example, with high measurement sensitivity and excellent responsibility and is hardly influenced by moisture or the like, for implementing precise sensing with small aged deterioration of measurement accuracy. Further, the inventive gas sensor employing the solid electrolyte is not only superior in energy saving to a conventional gas sensor employing a bulk sintered body but also operable at a low temperature, particularly at the room temperature, with no requirement for heating, whereby an excellent energy-saving effect can be attained.

In the gas sensor according to the present invention, the lithium ionic conductor constituting the said solid electrolyte preferably includes La_{(2/3)-X}Li_{3X}TiO₃ (0.05 ≤ X ≤ 0.15). In this case, La_{(2/3)-X}Li_{3X}TiO₃ constituting the solid electrolyte is preferably crystalline, and preferably has a columnar crystal structure or is amorphous.

In the gas sensor according to the present invention, the said solid electrolyte is preferably a filmy substance having a thickness of 1 to 3 µm.

The lithium ionic conductor constituting the said reference material in the gas sensor according to the present invention is preferably at least a material selected from a group consisting of lithium cobaltate, lithium nickelate, lithium manganate and lithium iron phosphate.

The said sensing material in the gas sensor according to the present invention is preferably characterized by any of the following (A) to (C):
(A) The sensing material is mainly composed of lithium carbonate, and further contains at least a carbonate of an alkaline earth metal.
(B) The sensing material is mainly composed of lithium nitrate, and further contains at least a nitrate of an alkaline earth metal.
(C) The sensing material is mainly composed of lithium sulfate, and further contains at least a sulfate of an alkaline earth metal.

The said sensing material in the gas sensor according to the present invention preferably further contains at least a material selected from a group consisting of lithium phosphate, phosphoryl lithium nitride and lithium titanate.

The present invention also provides a method of manufacturing a gas sensor comprising a structure including a reference pole having a reference material and an electronic conductive material and a sensing pole having a sensing material and an electronic conductive material provided through a solid electrolyte with the reference material, the sensing material and the solid electrolyte constituted of lithium ionic conductors and with the solid electrolyte containing La_{(2/3)-X}Li_{3X}TiO₃ (0.05 ≤ X ≤ 0.15) as the lithium ionic conductor, including the steps of forming the solid electrolyte, forming the reference pole and forming the sensing pole, in which the said step of forming the solid electrolyte is a step of forming a film of La_{(2/3)-X}Li_{3X}TiO₃ under an atmosphere sufficiently supplied with oxygen.

The present invention further provides a method of manufacturing a gas sensor comprising a structure including a reference pole having a reference material and an electronic conductive material and a sensing pole having a sensing material and an electronic conductive material provided through a solid electrolyte with the reference material, the sensing material and the solid electrolyte constituted of lithium ionic conductors and with the solid electrolyte containing La_{(2/3)-X}Li_{3X}TiO₃ (0.05 ≤ X ≤ 0.15) as the lithium ionic conductor, including the steps of forming the solid electrolyte, forming the reference pole and forming the sensing pole, in which the said step of forming the solid electrolyte is a step of forming a film of La_{(2/3)-X}Li_{3X}TiO₃ under a dry atmosphere having a dew point of not more than -40°C, and the steps for completely forming the reference pole and completely forming the sensing pole after forming the said solid electrolyte are carried out under the dry atmosphere having the dew point of not more than -40°C.

According to the inventive method of manufacturing a gas sensor, a gas sensor including a solid electrolyte having a high transport number of lithium ions can be manufactured, and a gas sensor having higher gas sensitivity and higher age-based stability can be obtained.

Particularly when the said step of forming the solid electrolyte is the step of forming a film of La_{(2/3)-X}Li_{3X}TiO₃ under a dry atmosphere having a dew point of not more than -40°C and the steps for completely forming the reference pole and completely the sensing pole after forming the said solid electrolyte are carried out under the dry atmosphere having the dew point of not more than -40°C, reduction of lithium ionic conductivity resulting from formation of an amorphous reaction layer can be prevented and a gas sensor stably exhibiting higher measurement sensitivity and superior responsibility also in a low-temperature range close to the room temperature can be manufactured.

The foregoing and other objects, features, aspects and advantages of the present invention will become more apparent from the following detailed description of the present invention when taken in conjunction with the accompanying drawings.

### BRIEF DESCRIPTION OF THE DRAWINGS

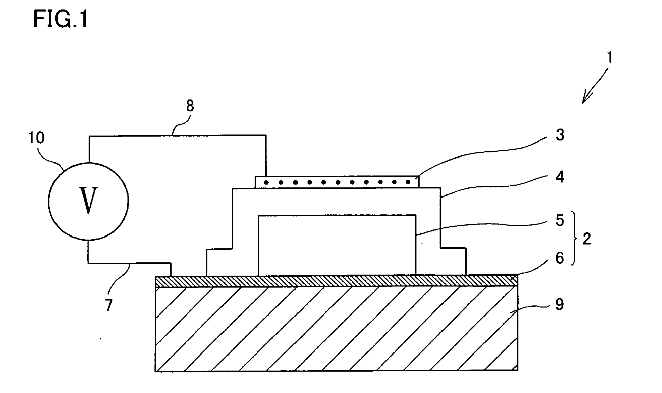

Fig. 1 is a sectional view schematically showing a gas sensor 1 according to a preferred embodiment of the present invention.

### DESCRIPTION OF THE PREFERRED EMBODIMENTS

Fig. 1 is a sectional view schematically showing a gas sensor 1 according to a preferred embodiment of the present invention. Gas sensor 1 according to the present invention comprises a structure including a reference pole 2 having a reference material and an electronic conductive material and a sensing pole 3 having a sensing material and an electronic conductive material provided through a solid electrolyte 4. The wording "provided through solid electrolyte 4" means that reference pole 4, sensing pole 3 and solid electrolyte 4 are so arranged that lithium ions move between reference pole 2 and sensing pole 3 through solid electrolyte 4. In other words, reference pole 2 and sensing pole 3 may be provided adjacently to surfaces opposite to each other in relation to the thickness direction of solid electrolyte 4 respectively as shown in Fig. 1, or may be provided adjacently to the same surface in relation to the thickness direction of solid electrolyte 4 in gas sensor 1 according to the present invention.

The feature of gas sensor 1 according to the present invention resides in that the said solid electrolyte 4, the said reference material and the said sensing material are constituted of lithium ionic conductors in the aforementioned structure. The term "lithium ionic conductor" indicates a solid capable of conducting lithium ions, which is not particularly restricted so far as the same has this property.

In gas sensor 1 according to the present invention, lithium ions are desorbed and electrons are discharged from the lithium ionic conductor constituting the reference material in reference pole 2, and the desorbed lithium ions are supplied to the sensing material contained in sensing pole 3 through solid electrolyte 4. Sensing pole 3 is supplied with lithium ions and electrons from the sensing material or solid electrolyte 4 and from the electronic conductive material contained in sensing pole 3 respectively, so that the lithium ions and the electrons react with gas under the atmosphere.

More specifically, lithium ions are desorbed and electrons are discharged from lithium cobaltate (LiCoO₂) through reaction expressed as LiCoO₂ = _{y}Li⁺ + _{y}e⁻ + Li_{1-y}CoO₂ if lithium cobaltate is employed as the lithium ionic conductor constituting the reference material in reference pole 2, for example. In the above expression, y represents the conversion rate (dissociation rate) of lithium to ions. At this time, the valence number of partial cobalt atoms constituting lithium cobaltate is oxidized from trivalence to tetravalence for balancing charges.

The lithium ions desorbed from reference pole 2 are supplied to the sensing material of sensing pole 3 through solid electrolyte 4. Sensing pole 3 is supplied with the lithium ions and the electrons from the sensing material or solid electrolyte 4 and from the electronic conductive material contained in sensing pole 3 respectively, so that the lithium ions and the electrons react with carbon dioxide and oxygen contained in the atmosphere to form lithium carbonate (Li₂ CO₃ if gas sensor 1 according to the present invention is a carbon dioxide sensor, for example. In other words, chemical reaction expressed as 2Li⁺ + CO₂ + (1/2)O₂⁺ 2e = Li₂ CO₃ progresses.

As a result of the aforementioned reaction in sensing pole 3 and reference pole 2, electromotive force E is generated between reference pole 2 and sensing pole 3. This electromotive force E follows the Nernst equation [E = E₀ + (RT/2F)ln(P_{CO2})] (where Eo represents a constant, R represents a gas constant, T represents the absolute temperature (K) of gas sensor 1, F represents the Faraday constant and P_{CO2} represents the carbon diode concentration. The oxygen concentration (P_{O2}) in the air is regarded as constant) in relation to the carbon dioxide concentration, whereby the carbon dioxide concentration in the air can be measured by measuring the quantity of electromotive force E. Gas sensor 1 can also measure the concentration of another gas such as nitrogen oxide gas or sulfur oxide gas through a similar function.

In gas sensor 1 according to the present invention, all of the reference material, solid electrolyte 4 and the sensing material are constituted of lithium ionic conductors as hereinabove described, whereby all conductive ions on the interfaces between reference pole 2, solid electrolyte 4 and sensing pole 3 constituting gas sensor 1 and in solid electrolyte 4 are lithium ions in the aforementioned reaction. Consequently, problems such as formation of an ion-exchange layer, occurrence of noise and aged deterioration are suppressed dissimilarly to a case of employing different types of ions. Further, the lithium ions are so excellently conductive that gas sensor 1 can precisely measure the gas concentration at a low temperature and attains high output responsibility. Age-based stabilization of the sensor output can be further improved by stabilizing flows of the lithium ions under direct current.

In gas sensor 1 according to the present invention, solid electrolyte 4, constituted of a lithium ionic conductor as hereinabove described, is implemented by a filmy substance formed by a dense sintered body of the lithium ionic conductor, for example. While the material for the lithium ionic conductor constituting solid electrolyte 4 can be prepared from La_{(2/3)-X}Li_{3X}TiO₃ (0.05 ≤ X ≤ 0.15), Li₄Ti₅O₁₂, lithium phosphorus oxynitride, LiTi₂(PO₄)₃ or Li₁₄Zn(GeO₄)₄, for example, and is not restricted in particular, La_{(2/3)-X}Li_{3X}TiO₃ (0.05 ≤ X ≤ 0.15) is preferably employed in consideration of particularly excellent ionic conductivity attained through this material. If X is less than 0.05 or in excess of 0.15 in the aforementioned chemical formula, the ionic conductivity tends to lower.

If solid electrolyte 4 is constituted of La_{(2/3)-X}Li_{3X}TiO₃ in gas sensor 1 according to the present invention, this La_{(2/3)-X}Li_{3X}TiO₃ is preferably crystalline, and preferably has a columnar crystal structure. The term "crystalline" indicates a state having such crystallinity that a peak is observed by X-ray diffraction with a thin-film X-ray diffractometer, for example. The term "columnar crystal structure" indicates a structure provided with columnar crystals extending in a thickness direction, and formation of this structure can be confirmed through observation with a scanning electron microscope (SEM), for example.

High lithium ionic conductivity of at least 10⁻⁴ S/cm can be attained at the room temperature through measurement by a direct current method (measurement method employing direct current) by employing solid electrolyte 4 prepared from the lithium ionic conductor of crystalline La_{(2/3)-X}Li_{3X}TiO₃ having a columnar crystal structure. Consequently, gas sensor 1 is further improved in output responsibility and sensitivity. The number of grain boundaries hindering ionic conduction is conceivably reduced due to the columnar crystal structure of La_{(2/3)-X}Li_{3X}TiO₃ contained in solid electrolyte 4, so that gas sensor 1 attains excellent characteristics.

While La_{(2/3)-X}Li_{3X}TiO₃ having a perovskite crystal structure is also known as a lithium ionic conductor having high ionic conductivity (refer to Japanese Patent Laying-Open No. 7-169456), this ionic conductivity is expressed in an intragranular value measured by an alternating current method (complex impedance measurement method (impedance spectroscopy)), and DC ionic conductivity is not more than the level of 10⁻⁶ S/cm in a generally used polycrystalline material such as a sintered body in practice due to high grain boundary resistance.

If solid electrolyte 4 of gas sensor 1 according to the present invention is constituted of La(_{2/3})_{-X}Li_{3X}TiO₃, high ionic conductivity can be attained also when La_{(2/3)-X}Li_{3X}TiO₃ is amorphous. The term "amorphous" indicates such a state that no peak is observed through X-ray diffraction. In this case, the amorphous La_{(2/3)-X}Li_{3X}TiO₃ having no grain boundaries exhibits no grain boundary resistance, whereby high lithium ionic conductivity exceeding the level of 10⁻³ S/cm can be attained not only in measurement by the aforementioned alternating current method but also in measurement by the aforementioned direct current method.

While the O (oxygen) content is set to 3 in the chemical formula La_{(2/3)-X}Li_{3X}TiO₃ according to the custom of notation of the perovskite crystal structure, appropriate oxygen deficiency results from compositional deviation of La and Li in practice.

Solid electrolyte 4, which may be constituted of only pure La_{(2/3)-X}Li_{3X}TiO₃ (0.05 ≤ X ≤ 0.15), may also contain another component such as a matric structure (polymer such as polyethylene, for example) in addition to La_{(2/3)-X}Li_{3X}TiO₃, as a matter of course.

When solid electrolyte 4 of gas sensor 1 according to the present invention is implemented as a filmy substance (solid electrolytic film) as shown in Fig. 1, the thickness thereof is preferably small so that DC resistance is reduced and the performance of gas sensor 1 can be further improved. The solid electrolytic film is preferably so implemented as to have a thickness of not more than 3 µm, in order to attain practical responsibility and practical measurement accuracy. In order to prevent sensing pole 3 and reference pole 2 from a short circuit resulting from pinholes formed in the solid electrolytic film, the solid electrolytic film is preferably so implemented as to have a thickness of at least 1 µm.

Reference pole 2 of gas sensor 1 according to the present invention has the reference material and the electronic conductive material. Reference pole 2 is preferably constituted of a filmy substance (reference material film 5) formed by the reference material and another filmy substance (electronic conductive material film 6), formed by the electronic conductive material, in contact with reference material film 5, as shown in Fig. 1. In this case, gas sensor 1 is preferably so implemented that reference material film 5 is in contact with the filmy substance (solid electrolytic film) of solid electrolyte 4 while electronic conductive material film 6 is in contact with a wire 7 as shown in Fig. 1, for supplying electrons generated in the reference material constituting reference material film 5 to sensing pole 3 through wires 7 and 8.

In gas sensor 1 according to the present invention, the reference material contained in reference pole 2 (contained in reference material film 5 in the embodiment shown in Fig. 1) is constituted of a lithium ionic conductor, as hereinabove described. The lithium ionic conductor constituting the reference material, not particularly restricted, is preferably prepared from a lithium-containing composite oxide composed of at least a material selected from a group consisting of transient metal-containing oxides such as lithium cobaltate (LiCoO₂), lithium nickelate (LiNiO₂) and lithium iron phosphate (LiFePO₄) and lithium manganate (LiMn₂O₄). An excellent interface can be formed between the reference material of such a lithium-containing composite oxide and the lithium ionic conductor (preferably La_{(2/3)-X}Li_{3X}TiO₃) constituting solid electrolyte 4, so that the interfacial resistance of lithium ions can be reduced to a practically satisfactory level under direct current.

Reference material film 5 preferably contains a crystalline reference material, and the thickness thereof is preferably at least 6 µm. The term "crystalline reference material" indicates a reference material having such crystallinity that a peak is observed by X-ray diffraction with a thin-film X-ray diffractometer, for example, similarly to the aforementioned case of solid electrolyte 4. The thickness of reference material film 5 is so set to at least 6 µm that gas sensor 1 can be sufficiently continuously used for about 10 years due to a sufficient lithium content (lithium capacity).

While the thickness of reference material film 5 relates to the life and age-based stability of gas sensor 1, the quantity of lithium ions dischargeable from a reference material prepared from lithium cobaltate, for example, is about 0.15 Ah/g (ampere hours/gram), and about 0.07 Ah/g (about half the quantity 0.15 Ah/g) of lithium ions can be practically used in consideration of practical stability of the output voltage of gas sensor 1 in a driven state. If reference material film 5 has a thickness of 6 µm in gas sensor 1 of 2 mm square, therefore, gas sensor 1 can be continuously driven for about 10 years assuming that the value of direct current flowing therein in equilibrium potential measurement is 100 pA (picoamperes). The abbreviation AH/g expresses the quantity of ions, allowing feeding of electricity for one hour at a certain current value, per unit weight. Also when reference material film 5 is prepared from lithium nickelate or lithium manganate, gas sensor 1 can be sufficiently continuously used for about 10 years, depending on the specific weight, the type of utilizable lithium and the like in practice, if the thickness of reference material film 5 is at least 6 µm.

The reference material employed in the present invention is preferably crystalline, as hereinabove described. Lithium ions easily flow in the reference material due to the crystallinity thereof. While each of lithium cobaltate and lithium nickelate has the so-called layered crystal structure, a and b axes in this layered crystal structure are more preferably oriented as perpendicularly as possible to the surface of reference material film 5 in consideration of flowability of lithium ions. In gas sensor 1 according to the present invention, however, the quantity of the actually flowing current is about 100 pA (picoamperes). Therefore, the a and b axes of the reference material forming reference material film 5 may alternatively be horizontally oriented or may be oriented at random, so that gas sensor 1 is practically employable.

The electronic conductive material constituting reference pole 2 is prepared from gold, platinum, palladium, rhodium, ruthenium or iridium in consideration of long-term corrosion resistance or the like, while aluminum, copper, nickel, iron or stainless steel is also employable. However, the electronic conductive material is preferably prepared from a metal having a catalytic effect, and a metal alloyed with the ionic conductor such as lithium is unusable. Among the aforementioned materials, platinum having stability with respect to lithium ions is preferably employed as the electronic conductive material.

Sensing pole 3 in gas sensor 1 according to the present invention has the sensing material and the electronic conductive material. While a lithium ionic conductor is employed for the sensing material in the present invention as hereinabove described, this lithium ionic conductor can be prepared from any lithium salt in response to the species of the sensed gas. While the sensing material can be prepared from only the lithium salt, salt of another alkaline earth metal is preferably also employed in response to the species of the sensed gas, in order to improve moisture resistance.

When gas sensor 1 according to the present invention is employed for sensing carbon dioxide, for example, a sensing material mainly composed of lithium carbonate along with at least a carbonate of an alkaline earth metal can be preferably employed. Strontium, calcium etc. can be listed as examples of the alkaline earth metal. If the sensing material contains such carbonate by 30 to 50 % in molar fraction, moisture resistance is improved.

In this case, sensing pole 3 of gas sensor 1 is supplied with lithium ions and electrons from the sensing material or solid electrolyte 4 and from the electronic conductive material respectively, so that the lithium ions and the electrons react with carbon dioxide and oxygen contained in the atmosphere to form lithium carbonate. Electromotive force is generated in response to the carbon dioxide concentration due to this reaction, so that gas sensor 1 can sense the carbon dioxide.

The inventors have found out that an excellent gas sensor capable of sensing gas of a nitrogen oxide or a sulfur oxide can be implemented when a nitrate or a sulfate of lithium is employed as the lithium ionic conductor constituting the sensing material contained in sensing pole 3 in place of the carbonate of lithium.

When gas sensor 1 according to the present invention is employed for sensing nitrogen oxide gas, for example, a sensing material mainly composed of lithium nitrate along with at least a nitrate of an alkaline earth metal can be preferably employed. Also in this case, sensing pole 3 is supplied with lithium ions and electrons from the sensing material or solid electrolyte 4 and from the electronic conductive material respectively, so that the lithium ions and the electrons react with nitrogen oxide gas and oxygen contained in the atmosphere to form lithium nitrate. Gas sensor 1 can sense nitrogen oxide gas due to this reaction. Also in this case, moisture resistance can be improved by employing the nitrate of the alkaline earth metal such as strontium or calcium for the sensing material.

When gas sensor 1 according to the present invention is employed for sensing sulfur oxide gas, for example, a sensing material mainly composed of lithium sulfate along with at least a sulfate of an alkaline earth metal can be preferably employed. Also in this case, sensing pole 3 is supplied with lithium ions and electrons from the sensing material or solid electrolyte 4 and from the electronic conductive material respectively, so that the lithium ions and the electrons react with sulfur oxide gas and oxygen contained in the atmosphere to form lithium sulfate. Gas sensor 1 can sense sulfur oxide gas due to this reaction. Also in this case, moisture resistance can be improved by employing the sulfate of the alkaline earth metal such as strontium or calcium for the sensing material.

In gas sensor 1 according to the present invention, at least one lithium compound selected from a group consisting of lithium phosphate (Li₃PO₄), phosphoryl lithium nitride (Li₍₃₊₁₎PO₍₄₋ₘ₎Nₙ, 0 < 1, m, n< 1) and lithium titanate (Li₄Ti₅O₁₂) is preferably mixed into the sensing material. When such a lithium compound is mixed into the sensing material, lithium ionic conductivity of the sensing material is so improved that gas sensor 1 can be implemented with a higher speed of response.

In gas sensor 1 according to the present invention, sensing pole 3 is preferably formed by a filmy substance of the aforementioned sensing material. In this case, the thickness of the filmy substance (sensing material film) of the sensing material is preferably not more than 1 µm. If gas sensor 1 according to the present invention is employed for sensing carbon dioxide, for example, high responsibility can be attained on the order of seconds at a temperature of not more than 200°C or even at the room temperature by setting the thickness of the sensing material film to not more than 1 µm, although the ionic conductivity of lithium carbonate lowers at a temperature of not more than 200°C. In order to attain a higher speed of response, the thickness of the sensing material film is more preferably not more than 0.5 µm. If gas sensor 1 according to the present invention comprises an additional heater (not shown) for performing measurement by heating sensing pole 3 with this heater, however, the thickness of the sensing material film may exceed 1 µm.

The electronic conductive material contained in sensing pole 3 of gas sensor 1 according to the present invention can be prepared from a material similar to that described above as the electronic conductive material contained in reference pole 2. In order to attain excellent responsibility, sensing pole 3 is preferably implemented by a structure in which the electronic conductive material and the sensing material are electrically integrated with each other. The wording "electronically integrated with each other" means such a state that the electronic conductive material and the sensing material are adhesively bonded to each other. More specifically, the electronic conductive material and the sensing material are preferably in contact with each other on a wide interface in sensing pole 3, so that lithium ions, the gas species to be measured, oxygen and electrons are efficiently supplied to the interface to smoothly cause reaction therebetween.

This structure is preferably implemented by a filmy substance (electronic conductive material film) of the electronic conductive material forming a fine network along with the said sensing material film. More specifically, the following three structures are preferably illustrated:
(a) Such a structure that the sensing material film is formed on the solid electrolytic film and the network of the electronic conductive material film is formed thereon.
   In the case of this structure, the electronic conductive material film is so formed as not to completely cover the sensing material film but to cover the sensing material film while partially exposing the same. The area of the part of the sensing material film exposed from the electronic conductive material film is preferably in the range of 15 to 85 % of the overall area of the sensing material film. If the area of the exposed part is less than 15 % of the overall area of the sensing material film, supply of the gas species and oxygen to the sensing material may be limited to result in insufficient measurement sensitivity. If the area of the exposed part exceeds 85 % of the overall area of the sensing material film, on the other hand, the network of the electronic conductive material film may be easily partially cut to result in reduction of measurement sensitivity due to formation of parts supplied with no electrons.
(b) Such a structure that the network of the electronic conductive material film is formed on the solid electrolytic film and the sensing material film is formed thereon.
   In the case of this structure, the electronic conductive material film is so formed as not to completely cover the solid electrolytic film but to cover the solid electrolytic film while partially exposing the same. The area of the part of the solid electrolytic film exposed from the electronic conductive material film is preferably in the range of 15 to 85 % of the overall area of the solid electrolytic film. If the area of the exposed part is less than 15 % of the overall area of the solid electrolytic film, supply of lithium ions from the solid electrolytic film may be rate-limited to result in insufficient measurement sensitivity. If the area of the exposed part exceeds 85 % of the overall area of the solid electrolytic film, on the other hand, the network of the electronic conductive material film may be easily partially cut to result in reduction of measurement sensitivity due to formation of parts supplied with no electrons. While the sensing material film covers the solid electrolytic film and the electronic conductive material film in this case, the sensed gas species and oxygen are supplied to the electronic conductive material film through pores inevitably formed in the sensing material film.
(c) Such a structure that the electronic conductive material film and the sensing material film are mixedly provided on the solid electrolytic film to form the network of the electronic conductive material film while the solid electrolytic film is exposed from under the electronic conductive material film and the sensing material film (shown in Fig. 1).
   In the case of this structure, the electronic conductive material film and the sensing material film are mixedly integrated with each other to form sensing pole 3. The area ratio between the electronic conductive material film and the sensing material film in sensing pole 3 is preferably in the range of 15:85 to 85:15. The network of the electronic conductive material film tends to be easily partially cut if the area ratio of the electronic conductive material film is less than 15 %, and exhibits a similar tendency also when this area ratio exceeds 85 %. While the electronic conductive material film and the sensing material film cover the solid electrolytic film while partially exposing the same in this structure, the area of the exposed part of the solid electrolytic film is preferably not more than 85 % of the overall area of the solid electrolytic film. If the area of the exposed part exceeds 85 % of the overall area of the solid electrolytic film, the number of regions where the network of the electronic conductive material film is cut may be increased while the number of regions of the sensing material film reacting with the gas species and oxygen may be reduced to result in insufficient measurement sensitivity.

In gas sensor 1 according to the present invention, the aforementioned reference pole 2, solid electrolyte 4 and sensing pole 3 are preferably formed on a substrate 9 holding the same, as shown in Fig. 1. The material for substrate 9, not particularly restricted, is preferably prepared from quartz glass, sapphire, strontium titanate, aluminum nitride, silicon nitride or magnesium oxide. A heating source (not shown) such as a platinum heater may be formed on a region of the back surface of substrate 9 corresponding to the region of the front surface formed with reference pole 2, solid electrolyte 4 and sensing pole 3. In this case, the heating source can be formed by screen-printing platinum paste onto substrate 9 and baking the same before forming gas sensor 1.

Gas sensor 1 according to the present invention can be manufactured by a method having the steps of forming solid electrolyte 4, forming reference pole 2 and forming sensing pole 3. For example, gas sensor 1 can be manufactured by forming reference pole 2 on substrate 9 and further forming solid electrolyte 4 and sensing pole 3. Reference material film 5 and electronic conductive material film 6 constituting reference pole 2 and the filmy substance (solid electrolytic film) of solid electrolyte 4 can be formed by a vapor phase method, for example. While reference material film 5, electronic conductive material film 6 and the solid electrolytic film can be formed by a vapor phase method such as vapor deposition, ion plating, sputtering or laser ablation, the method is not restricted to these. Alternatively, reference material film 5, electronic conductive material film 6 and the solid electrolytic film can be formed by a sol-gel process employing a metal organic compound as a raw material.

When gas sensor 1 according to the present invention is employed for sensing carbon dioxide, for example, the sensing material film can be formed by a vapor phase method such as vapor deposition, ion plating, sputtering or laser ablation with a starting material formed by a carbooxide of an alkaline earth metal such as lithium carbonate or strontium carbonate.

The sensing material film can alternatively be prepared through gas deposition by spraying raw material powder of a solid state onto substrate 9 through a gas jet. The sensing material film can further alternatively be prepared by separately or simultaneously forming films of a lithium metal and an alkaline earth metal or films of lithium oxide and strontium oxide in metallic states by a vapor phase method and thereafter reacting the films with moistened carbon dioxide. Also when gas sensor 1 according to the present invention is employed for another sensing gas (nitrogen oxide gas or sulfur oxide gas, for example), the sensing material film can be prepared similarly to the above, except that carbonate and carbon dioxide are replaced with raw materials corresponding to this gas.

The electronic conductive material film constituting sensing pole 3 can be formed through pattern formation utilizing a mask. For example, an electronic conductive material film having a prescribed thickness may be formed by this method with a prescribed area ratio, so that a sensing material film having a prescribed thickness is formed on the surface thereof.

The electronic conductive material film and the sensing material film may be simultaneously formed. The ratio between the areas of the electronic conductive material film and the sensing material film can be adjusted by controlling the yields of gaseous raw materials therefor when a vapor phase method is employed, or by controlling the mixing ratio between the raw powder materials or the gas flow rates of nozzles for the raw powder materials when gas deposition is employed. Alternatively, the electronic conductive material film may be first provided in the form of an island having a thickness of several nm (nanometers) to several 10 nm so that the electronic conductive material film and the sensing material film are simultaneously formed by the aforementioned method.

The electronic conductive material film and the sensing material film may be formed by applying a pasty mixture of sensing material powder and electronic conductive material powder and thereafter baking the same. Alternatively, the electronic conductive material film and the sensing material film may be formed by a sol-gel process while employing a metal organic compound as the raw material for the sensing material.

The present invention provides a method of manufacturing the aforementioned gas sensor 1 according to the present invention, in which the lithium ionic conductor constituting solid electrolyte 4 is La_{(2/3)-X}Li_{3X}TiO₃ (0.05 ≤ X ≤ 0.15) in particular.

The method of manufacturing a gas sensor according to an aspect of the present invention is a method of manufacturing gas sensor 1 comprising a structure including reference pole 2 having a reference material and an electronic conductive material and sensing pole 3 having a sensing material and an electronic conductive material provided through solid electrolyte 4 with the reference material, the sensing material and solid electrolyte 4 constituted of lithium ionic conductors and with solid electrolyte 4 containing La_{(2/3)-X}Li_{3X}TiO₃ (0.05 ≤ X ≤ 0.15) as the lithium ionic conductor, including the steps of forming solid electrolyte 4, forming reference pole 2 and forming sensing pole 3, in which the said step of forming solid electrolyte 4 is a step of forming a film of La_{(2/3)-X}Li_{3X}TiO₃ under an atmosphere sufficiently supplied with oxygen.

In an ionic conductor of an oxide, oxygen deficiency generally causes reduction of electronic conductivity. The transport number of lithium ions (proportion of lithium ionic conductivity in total electronic conductivity) is reduced due to this reduction of the electronic conductivity, not only to cause reduction of gas sensitivity but also to result in age-based instability. The inventor has conducted deep studies, to find out that the film of La_{(2/3)-X}Li_{3X}TiO₃ is preferably formed under an atmosphere sufficiently supplied with oxygen in formation of solid electrolyte 4, in order to set the transport number of lithium ions to at least 0.9 by reducing the degree of oxygen deficiency causing reduction of the electronic conductivity. In the aforementioned method of manufacturing a gas sensor according to the present invention, the film of La_{(2/3)-X}Li_{3X}TiO₃ constituting solid electrolyte 4 is formed under an atmosphere capable of sufficiently supplying oxygen, whereby oxygen deficiency causing reduction of the electronic conductivity can be rendered restrictive so that gas sensor 1, having a lithium ion transport number of at least 0.9, improved in sensitivity and stabilized in output can be preferably manufactured.

The wording "atmosphere sufficiently supplied with oxygen" indicates an atmosphere capable of supplying oxygen to such a degree that the oxygen content in formed solid electrolyte 4 can reach a stoichiometric composition. This atmosphere can be sufficiently implemented when the oxygen partial pressure thereof is about 10⁻³ Torr, if solid electrolyte 4 is formed by laser ablation, for example. The aforementioned atmosphere capable of sufficiently supplying oxygen can be substantially similarly implemented by another vapor phase method such as vapor deposition, ion plating or sputtering, for example. The optimum oxygen partial pressure can be obtained by conducting a preliminary experiment with various oxygen partial pressures, for controlling the oxygen partial pressure of the atmosphere.

The present invention also provides a method of manufacturing gas sensor 1 comprising a structure including reference pole 2 having a reference material and an electronic conductive material and sensing pole 3 having a sensing material and an electronic conductive material provided through solid electrolyte 4 with the reference material, the sensing material and solid electrolyte 4 constituted of lithium ionic conductors and with solid electrolyte 4 containing La_{(2/3)-X}Li_{3X}TiO₃ (0.05 ≤ X ≤ 0.15) as the lithium ionic conductor, including the steps of forming solid electrolyte 4, forming reference pole 2 and forming sensing pole 3, in which the said step of forming solid electrolyte 4 is a step of forming a film of La_{(2/3)-X}Li_{3X}TiO₃ under a dry atmosphere having a dew point of not more than -40°C, and the steps for completely forming reference pole 2 and completely forming sensing pole 3 after forming solid electrolyte 4 are carried out under the dry atmosphere having the dew point of not more than -40°C.

In the process of studying the characteristics, particularly chemical stability, of a filmy substance prepared from La_{(2/3)-X}Li_{3X}TiO₃, the inventors have found out that this filmy substance may react with moisture or carbon dioxide contained in the air to form an amorphous reaction layer on a portion, having a thickness of 0.1µm, of the surface thereof. This amorphous reaction layer, so stable that the same causes no further reaction upon formation, may remarkably reduce Li ionic conductivity to influence the performance of the gas sensor.

The inventors have further studied a method of preventing formation of this amorphous reaction layer, to find out that all steps, including the steps of forming the solid electrolytic film and forming reference pole 2 and/or sensing pole 3 as well as transfer and preservation steps possibly inserted between the aforementioned steps, must be so carried out that the filmy substance of La_{(2/3)-X}Li_{3X}TiO₃ is not in contact with an atmosphere having a dew point exceeding -40°C when the filmy substance (solid electrolytic film) of La_{(2/3)-X}Li_{3X}TiO₃ and/or sensing pole 3 is formed.

More specifically, apparatuses for forming the filmy substance of La_{(2/3)-X}Li_{3X}TiO₃ (0.05 ≤ X ≤ 0.15) and sensing electrode 3 respectively may be provided with pass boxes filled with gas and/or air having a dew point of not more than -40°C so that a closed vessel is employed for introducing/discharging a sample (gas sensor 1 in the process of manufacturing) into/from these apparatuses through the pass boxes, when gas sensor 1 is manufactured by first forming reference pole 2 on substrate 9, thereafter forming the filmy substance of La_{(2/3)-X}Li_{3X}TiO₃ and then forming sensing pole 3. Thus, sensing pole 3 can be formed with no amorphous reaction layer formed on the filmy substance of La_{(2/3)-X}Li_{3X}TiO₃. Consequently, Li ionic conductivity can be prevented from reduction resulting from formation of an amorphous reaction layer, whereby a gas sensor having excellent performance, particularly a gas sensor capable of stably sensing gas with high measurement sensitivity and excellent responsibility in a low temperature range close to the room temperature can be obtained.

While dry air is employed as the atmosphere having the dew point of not more than -40°C, inert gas such as argon is preferably employed in place of the air.

After completion of gas sensor 1 according to the present invention, solid electrolyte 4 containing La_{(2/3)-X}Li_{3X}TiO₃ may react with moisture or carbon dioxide contained in the atmosphere over a long period of several years to form an amorphous reaction layer on the surface thereof such that the Li ionic conductivity is remarkably reduced by the amorphous reaction layer to influence the performance of gas sensor 1. In order to improve long-term stability, therefore, gas sensor 1 according to the present invention may be implemented by such a structure (not shown) that solid electrolyte 4 is not in contact with the atmosphere (implemented by covering portions other than sensing pole 3 with a polymer layer for blocking the outside air, for example). Heat-resistant engineering plastics such as aromatic polyamide, polyimide or polyether sulfone can be properly selected as the material for the polymer layer in response to the working temperature thereof.

### Examples

The present invention is now described with reference to Examples. The present invention is not restricted to the following Examples, but various modifications can be applied in the range equivalent and equal to the present invention.

### (Example 1)

A filmy substance of gold having a thickness of 0.1 µm was formed on the overall surface of a quartz glass substrate 9 of 10 cm square having a thickness of 0.5 mm with linear notches vertically and horizontally formed on the back surface at intervals of 5 mm by vapor deposition, thereby forming an electronic conductive material film 6.

Then, a filmy substance of lithium cobaltate having a thickness of 6 µm was formed on a portion, having an area 2 mm square, of electronic conductive material film 6 corresponding to the central notch of 5 mm square provided on the back surface of substrate 9 by laser ablation through a mask, thereby forming a reference material film 5. More specifically, powder of lithium cobaltate molded into a pellet through a die was set in a vacuum vessel as a laser target while substrate 9 formed with electronic conductive film 6 was set on a substrate holder in the vacuum vessel. Then, a KrF excimer laser beam was applied to the target under an oxygen gas atmosphere of 10⁻³ Torr while controlling the temperature of the substrate holder to 700°C. Thus, a filmy substance of lithium cobaltate was formed on the gold filmy substance formed on substrate 9, thereby forming a reference material film 5. It was confirmed by thin-film X-ray diffraction that the filmy substance of lithium cobaltate was (1 10)-oriented. Thus, a reference pole 2 constituted of reference material film 5 and electronic conductive material film 6 was formed on substrate 9.

Then, a filmy substance (solid electrolytic film) of a solid electrolyte 4 was formed to cover reference material film 5. First, a solid electrolytic film containing La_{0.56}Li_{0.33}TiO₃ (corresponding to X = about 0.11 in La_{(2/3)-X}Li_{3X}TiO₃) was formed under conditions similar to those for the aforementioned filmy substance of lithium cobaltate except that a sintered body having the composition La_{0.56}Li_{0.33}TiO₃ was employed as a target along with a mask for forming a film having an area 3 mm square. The solid electrolytic film was so formed as to cover the filmy substance of lithium cobaltate constituting reference material film 5, so that the thickness thereof was 2 µm.

When a similar film was formed on a dummy substrate under the same conditions and the composition thereof was analyzed by inductively coupled plasma analysis (hereinafter abbreviated as ICP analysis), it was confirmed that the composition ratios between La, Li and Ti were substantially identical to those of the target La_{0.56}Li_{0.33}TiO₃, i. e., X = 0.11.

When another similar film was formed on another dummy substrate provided with a thin gold electrode film under the same conditions and another thin gold electrode film was formed thereon for measuring DC and AC electric conductivity in the thickness direction, the lithium ionic conductivity was 1 × 10⁻³ S/cm and the lithium ion transport number was 0.999.

When the obtained filmy substance of La_{0.56}Li_{0.}3₃TiO₃ was cut along with substrate 9 and the cut surface was observed with a scanning electron microscope (SEM), columnar crystals of 0.1 to 1 µm in width extended in the thickness direction to form a structure (columnar crystal structure). A diffraction peak corresponding to La_{0.56}Li_{0.33}TiO₃ crystals was observed also in thin-film X-ray diffraction. It was confirmable from these results that a preferable solid electrolytic film was formed.

A sensing pole 3 in which a filmy substance (sensing material film) of lithium carbonate serving as a sensing material and another filmy substance (electronic conductive material film) of gold serving as an electronic conductive material were mixedly integrated with each other was formed on this solid electrolytic film by laser ablation through a mask for forming a film 2 mm square. More specifically, a molding obtained by stamping a semicircular gold plate and lithium carbonate powder together in a die was used as a target. In other words, a surface of this target irradiated with a laser beam was equally composed of gold and lithium carbonate powder. The rotation velocity of the target was set to 2 rpm (revolutions per minute), and a film was formed by repetitively applying an excimer laser beam with a pulse of 5 Hz. The laser beam was first applied to gold. At this time, the film was formed at the room temperature with a degree of vacuum of 10⁻⁶ Torr for 30 minutes, thereby forming sensing pole 3 having a thickness of 0.5 µm.

The surface of the prepared sample was etched with an argon ion beam through a focused ion beam (FIB) apparatus, and a portion close to the interface between the sample and the thin film of La_{0.56}Li_{0.33}TiO₃ was observed with a transmission electron microscope (TEM). As a result, it was observed that filmy substances of gold and lithium carbonate of 5 to 10 nm in thickness were mosaically laminated. The ratio of the filmy substance of La_{0.56}Li_{0.33}TiO₃ covered with sensing pole 3 having the laminated filmy substances was estimated at about 20 %. It was confirmable from these results that sensing pole 3 was excellently formed. The thickness of each film was measured with a probe-type step meter.

Then, gold probes of 0.2 mm in diameter were electrically connected to the gold filmy substance (electronic conductive material film 6) of reference pole 2 and sensing pole 3 as wires 7 and 8 respectively. An electrometer 10 was electrically connected between wires 7 and 8. Thus, carbon dioxide sensor 1 according to the present invention was prepared as shown in Fig. 1.

A performance test of gas sensor 1 prepared in the aforementioned manner is now described. Standard gas having a carbon dioxide concentration of 350 ppm similarly to the atmosphere and another standard gas having a higher carbon dioxide concentration of 1000 ppm were prepared by adding carbon dioxide to a gas mixture of 22 % of oxygen and 78 % of nitrogen having relative humidity of 60 % at the room temperature. Carbon dioxide sensor 1 prepared in the aforementioned manner was introduced into a measurement vessel and connected to electrometer 10 having input impedance of 1 GΩ (gigaohm) for measuring outputs of carbon dioxide sensor 1 while alternately feeding the said standard gas having the carbon dioxide concentration of 350 ppm and that having the carbon dioxide concentration of 1000 ppm into the measurement vessel held at a temperature of 50°C. Consequently, outputs of -150 mV and -100 mV were measured in the cases of the carbon dioxide concentrations of 350 ppm and 1000 ppm respectively in a stable manner with respect to the different carbon dioxide concentrations.

Carbon dioxide sensor 1, requiring 20 seconds for stabilizing the output when fed with the gas having the higher carbon dioxide concentration and 40 seconds when fed with the gas having the lower carbon dioxide concentration, was proved to be excellent in responsibility. Influence of temperature fluctuation of gas sensor 1 resulting from change of the gas flow rate was corrected according to the Nernst equation on the basis of a temperature detected by a thermocouple attached to gas sensor 1.

Further, output change of gas sensor 1 measured by maintaining the carbon dioxide concentration and the temperature of the measurement vessel at 350 ppm and 50°C respectively over 350 days was within ±0.5 mV. Thus, gas sensor 1 was proved to be capable of stably measuring carbon dioxide over a long period with excellent age-based stability.

### (Examples 2 to 7)

Gas sensors according to Examples 2 to 7 were prepared under the same conditions as those for Example 1, except that the Li contents (X in the chemical formula La_{(2/3)-X}Li_{3X}TiO₃; shown in Table 1) of thin films of La_{(2/3)-X}Li_{3X}TiO₃ were varied for varying ionic conductivity levels as shown in Table 1, and lithium ion transport numbers and sensor outputs were measured under the same conditions as those for Example 1. Table 1 shows the results.

**Table 1**

| | La_{(2/3)-X}Li_{3X}TiO₃ Film | | | Sensor Output (mV) | |
|---|---|---|---|---|---|
| | X Value | Ionic conductivity (10⁻⁵ S/cm) | Lithium Ion Transport Number | Carbon Dioxide Concentration 350 ppm | Carbon Dioxide Concentration 1000 ppm |
| Example 2 | 0.05 | 10 | 0.999 | -100 | -75 |
| Example 3 | 0.08 | 40 | 0.999 | -150 | -100 |
| Example 4 | 0.15 | 30 | 0.999 | -140 | -95 |
| Example 5 | 0.04 | 8 | 0.999 | -60 | -45 |
| Example 6 | 0.16 | 9 | 0.999 | -85 | -50 |
| Example 7 | 0.20 | 4 | 0.999 | -30 | -25 |

From the results shown in Table 1, it is understood that the gas sensors according to Examples 2, 3 and 4 having the X values in the range of 0.05 to 0.15 exhibited high ionic conductivity levels and particularly high outputs.

### (Examples 8 to 10)

Gas sensors according to Examples 8 to 10 were prepared under the same conditions as those for Example 1 except that reference materials shown in Table 2 were employed, and outputs of these gas sensors were measured under the same conditions as those for Example 1. Table 2 shows the results. As understood from Table 2, the gas sensors obtained high outputs regardless of the types of the reference materials.

**Table 2**

| | Reference Material | Sensor Output (mV) | |
|---|---|---|---|
| | | Carbon Dioxide Concentration 350 ppm | Carbon Dioxide Concentration 1000 ppm |
| Example 8 | Lithium Nickelate (LiNiO₂) | -150 | -100 |
| Example 9 | Lithium Iron Phosphate (LiFePO₄) | -130 | -90 |
| Example 10 | Lithium Manganate (LiMn₂O₄) | -160 | -110 |

### (Examples 11 to 19)

Gas sensors according to Examples 11 to 19 were prepared under the same conditions as those for Example 1 except that sensing materials were prepared by adding 50 mole % of materials shown in Table 3 to lithium carbonate respectively, and outputs of these gas sensors were measured under the same conditions as those for Example 1. Table 3 shows the results. As understood from Table 3, the gas sensors obtained high outputs regardless of the types of the materials added to the sensing materials.

**Table 3**

| | Reference Material | Sensor Output (mV) | |
|---|---|---|---|
| | | Carbon Dioxide 350 ppm | Carbon Dioxide Concentration 1000 ppm |
| Example 11 | Strontium Carbonate (SrCO₃) | -150 | -100 |
| Example 12 | Barium Carbonate (BaCO₃) | -150 | -100 |
| Example 13 | Calcium Carbonate (CaCO₃) | -150 | -100 |
| Example 14 | Lithium Phosphate (Li₃PO₄) | -170 | -110 |
| Example 15 | Phosphoryl Lithium Nitride(Li_{3.3}PO₃₇N_{0.3}) | -170 | -110 |
| Example 16 | Lithium Titanate (Li₄Ti₅O₁₂) | -500 | -450 |
| Example 17 | Lithium Phosphate + Barium Carbonate (1:2) | -160 | -105 |
| Example 18 | Phosphoryl Lithium Nitride +Barium Carbonate (1:1) | -160 | -105 |
| Example 19 | Lithium Titanate +Barium Carbonate (1:3) | -350 | -300 |

### (Example 20)

A gas sensor according to Example 20 was prepared similarly to Example 1, except that lithium carbonate was replaced with lithium nitrate. Outputs of this gas sensor were measured under the same conditions as those for Example 1, except that nitrogen dioxide (NO₂) gas materials diluted to concentrations of 10 ppm and 100 ppm respectively with nitrogen gas were employed in place of the said standard gas of carbon dioxide. As a result, the gas sensor exhibited outputs of 0 mV, 20 mV and 60 mV for nitrogen gas containing no nitrogen dioxide, that containing 10 ppm of nitrogen dioxide and that containing 100 ppm of nitrogen dioxide respectively. With these results, the gas sensor obtaining sufficient outputs was confirmed to be capable of precisely sensing nitrogen dioxide.

### (Example 21)

A gas sensor according to Example 21 was prepared similarly to Example 1, except that lithium carbonate was replaced with lithium sulfate. Outputs of this gas sensor were measured under the same conditions as those for Example 1, except that sulfur dioxide (SO₂) gas materials diluted to concentrations of 10 ppm and 100 ppm respectively with sulfur gas were employed in place of the said standard gas of carbon dioxide. As a result, the gas sensor exhibited outputs of 0 mV, 10 mV and 30 mV for sulfur gas containing no sulfur dioxide, that containing 10 ppm of sulfur dioxide and that containing 100 ppm of sulfur dioxide respectively. With these results, the gas sensor obtaining sufficient outputs was confirmed to be capable of precisely sensing sulfur dioxide.

### (Example 22)

A gas sensor according to Example 22 was prepared by a method similar to that for manufacturing gas sensor 1 according to Example 1. First, a reference pole constituted of an electronic conductive material film and a reference material film was formed on a substrate, a filmy substance (solid electrolytic film) of La_{(2/3)-X}Li_{3X}TiO₃ (0.05 ≤ X ≤ 0.15) was formed on this reference material film, and a sensing pole constituted of a sensing material film and an electronic conductive material film integrated with each other was formed thereon.

Apparatuses for forming the solid electrolytic film and the sensing pole respectively were provided with pass boxes filled with inert gas such as argon or air having a dew point of not more than -40°C. Further, the sample was transferred and preserved in a closed vessel from formation of the solid electrolytic film up to complete formation of the sensing pole and introduced/discharged into/from the apparatuses through the pass boxes, not to be in contact with an atmosphere other than the inert gas such as argon or air having the dew point of not more than -40°C. Consequently, formation of an amorphous low ionic conduction layer (amorphous reaction layer) was suppressed on the film of La_{(2/3)-X}Li_{3X}TiO₃ (0.05 ≤ X ≤ 0.15) and Li ionic conductivity was improved, so that the gas sensor was capable of sensing gas at a lower temperature.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A gas sensor comprising a structure including a reference pole having a reference material and an electronic conductive material and a sensing pole having a sensing material and an electronic conductive material provided through a solid electrolyte, wherein
said solid electrolyte, said reference material and said sensing material are constituted of lithium ionic conductors.

2. The gas sensor according to claim 1, wherein
said lithium ionic conductor constituting said solid electrolyte includes La(_{2/3})-_{X}L_{13X}TiO₃ (0.05 ≤ X ≤ 0.15).

3. The gas sensor according to claim 2, wherein
La_{(2/3)-X}Li_{3X}TiO₃ constituting said solid electrolyte is crystalline, and has a columnar crystal structure.

4. The gas sensor according to claim 2, wherein
La_{(2/3)-X}Li_{3X}TiO₃ constituting said solid electrolyte is amorphous.

5. The gas sensor according to any one of the preceding claims, wherein
said solid electrolyte is a filmy substance having a thickness of 1 to 3 µm.

6. The gas sensor according to any one of the preceding claims, wherein
said lithium ionic conductor constituting said reference material is at least a material selected from a group consisting of lithium cobaltate, lithium nickelate, lithium manganate and lithium iron phosphate.

7. The gas sensor according to any one of the preceding claims, wherein
said sensing material is mainly composed of lithium carbonate, and further contains at least a carbonate of an alkaline earth metal.

8. The gas sensor according to claim 7, wherein
said sensing material further contains at least a material selected from a group consisting of lithium phosphate, phosphoryl lithium nitride and lithium titanate.

9. The gas sensor according to any one of claims 1 to 6, wherein
said sensing material is mainly composed of lithium nitrate, and further contains at least a nitrate of an alkaline earth metal.

10. The gas sensor according to claim 9, wherein
said sensing material further contains at least a material selected from a group consisting of lithium phosphate, phosphoryl lithium nitride and lithium titanate.

11. The gas sensor according to any one of claims 1 to 6, wherein
said sensing material is mainly composed of lithium sulfate, and further contains at least a sulfate of an alkaline earth metal.

12. The gas sensor according to claim 11, wherein
said sensing material further contains at least a material selected from a group consisting of lithium phosphate, phosphoryl lithium nitride and lithium titanate.

13. A method of manufacturing a gas sensor comprising a structure including a reference pole having a reference material and an electronic conductive material and a sensing pole having a sensing material and an electronic conductive material provided through a solid electrolyte with said reference material, said sensing material and said solid electrolyte constituted of lithium ionic conductors and with said solid electrolyte containing La_{(2/3)-X}Li_{3X}TiO₃ (0.05 ≤ X ≤ 0.15) as said lithium ionic conductor, including the steps of forming said solid electrolyte, forming said reference pole and forming said sensing pole, wherein
said step of forming said solid electrolyte is a step of forming a film of La_{(2/3)-X}Li_{3X}TiO₃ under an atmosphere sufficiently supplied with oxygen.

14. A method of manufacturing a gas sensor comprising a structure including a reference pole having a reference material and an electronic conductive material and a sensing pole having a sensing material and an electronic conductive material provided through a solid electrolyte with said reference material, said sensing material and said solid electrolyte constituted of lithium ionic conductors and with said solid electrolyte containing La_{(2/3)-X}Li_{3X}TiO₃ (0.05 ≤ X ≤ 0.15) as said lithium ionic conductor, including the steps of forming said solid electrolyte, forming said reference pole and forming said sensing pole, wherein
said step of forming said solid electrolyte is a step of forming a film of La_{(2/3)-X}Li_{3X}TiO₃ under a dry atmosphere having a dew point of not more than -40°C, and the steps for completely forming said reference pole and completely forming said sensing pole after forming said solid electrolyte are carried out under said dry atmosphere having said dew point of not more than -40°C.
